# EUROPEAN PATENT APPLICATION

(11) **EP 4 768 485 A1**
(43) Date of publication of application: **01.07.2026**
(21) Application number: 24874086.2
(22) Date of filing: 30.09.2024
(51) Int. Cl.: C07D 471/04, C07D 405/12, A61K 31/4353, A61P 31/04

(54) **FABI ENZYME INHIBITOR AND USE THEREOF**

(30) Priority: 07.10.2023 CN 202311290781
(71) Applicant: Guangzhou Baiyunshan Pharmaceutical Holdings Co., Ltd. Baiyunshan Pharmaceutical General Factory, Guangzhou, Guangdong 510515 (CN)
(72) Inventor: WANG, Jiansong, Guangzhou, Guangdong 510515 (CN); XIE, Zhoufan, Guangzhou, Guangdong 510515 (CN); QIN, Fei, Guangzhou, Guangdong 510515 (CN); LIU, Wentao, Guangzhou, Guangdong 510515 (CN); HU, Hairong, Guangzhou, Guangdong 510515 (CN); BAO, Yingxia, Guangzhou, Guangdong 510515 (CN)
(74) Representative: Guardian IP Consulting I/S
(86) International application number: PCT/CN2024/122861
(87) International publication number: WO 2025/073269

(57) **Abstract**

The present application discloses a FabI enzyme inhibitor and use thereof. The FabI enzyme inhibitor is a compound as represented by Formula I, provides a new option for clinical screening and/or preparation of drugs for the treatment of diseases associated with FabI enzyme activity.

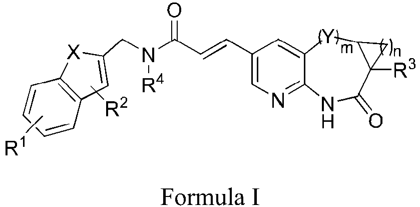

## Description

### TECHNICAL FIELD

The present disclosure relates to the field of pharmaceutical technology, in particular to a FabI enzyme inhibitor and use thereof.

### BACKGROUND

Fatty acid synthetase (FAS) is involved in an entire biosynthetic pathway of saturated fatty acids in all organisms. Each cycle of bacterial fatty acid biosynthesis includes four steps: step 1, condensation of malonyl-ACP with acetyl-CoA catalyzed by β-ketoacyl-ACP synthetase; step 2, reduction of ketoesters by a NADPH-dependent β-ketoacyl-ACP reductase; step 3, dehydration by β-hydroxyacyl-ACP dehydratase to obtain trans-2-enoyl-ACP; and step 4, conversion of trans-2-enoyl-ACP into acyl-ACP bearing two additional carbon atoms by NADH-dependent enoyl-ACP reductase (FabI). The cycle is repeated to obtain palmitoyl-ACP (C16). It can be seen that FabI enzyme is a key regulatory enzyme in the biosynthesis process of fatty acids. Therefore, by inhibiting the activity of FabI enzyme, the biosynthesis of bacterial fatty acids can be blocked, thereby inhibiting the growth and reproduction of bacteria, and playing a role in treating bacterial infections.

There are currently some compounds with FabI enzyme inhibitory activities, but as the number of patients affected by various bacterial infections and drug resistance increases, further development of new drugs for treating bacterial infections is still needed.

### SUMMARY

The present disclosure aims to address at least one of the aforementioned technical problems existing in the prior art. Therefore, an objective of the present disclosure is to provide a FabI enzyme inhibitor and use thereof.

In order to achieve the above objective, the technical solution of the present disclosure is provided as follows.

A first aspect of the present disclosure provides a compound of Formula I, or a stereoisomer, a deuterated compound, an isotopically labeled derivative, a pharmaceutically acceptable salt, a prodrug, or a solvate thereof: 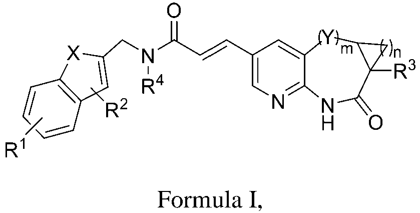
wherein X is selected from the group consisting of O, CR^{X1}R^{X2}, NR^{X1}, and S;
Y is selected from the group consisting of O, -CH₂-, NR^{Y}, -O-CH₂-, -CH₂-O-, -NR^{Y}-CH₂-, and -CH₂-NR^{Y}-;
R^{X1} and R^{X2} are each independently selected from the group consisting of hydrogen, -C₁₋₆ alkyl, and halogen-substituted -C₁₋₆ alkyl;
R^{Y} is selected from the group consisting of hydrogen, -C₁₋₆ alkyl, halogen-substituted -C₁₋₆ alkyl, -C₀₋₂ alkylene-C(O)R^{Y1}, -C₀₋₂ alkylene-C(O)NR^{Y1}R^{Y2}, -C₀₋₂ alkylene-NR^{Y1}R^{Y2}, -C₀₋₂ alkylene-NR^{Y1}C(O)R^{Y2}, -C₀₋₂ alkylene-(3-10-membered cycloalkyl), -C₀₋₂ alkylene-(3-10-membered heterocycloalkyl), -C₀₋₂ alkylene-(6-10-membered aromatic ring), and -C₀₋₂ alkylene-(5-10-membered heteroaromatic ring);
R^{Y1} and R^{Y2} are each independently selected from the group consisting of hydrogen, -C₁₋₆ alkyl, and halogen-substituted -C₁₋₆ alkyl;
R¹ is selected from the group consisting of hydrogen, halogen, cyano, nitro, -C₁₋₆ alkyl, halogen-substituted -C₁₋₆ alkyl, -C₀₋₂ alkylene-OR¹¹, -C₀₋₂ alkylene-C(O)R¹¹, -C₀₋₂ alkylene-C(O)NR¹¹R¹², -C₀₋₂ alkylene-NR¹¹R¹², -C₀₋₂ alkylene-NR¹¹C(O)R¹², -C₀₋₂ alkylene-(3-10-membered cycloalkyl), -C₀₋₂ alkylene-(3-10-membered heterocycloalkyl), -C₀₋₂ alkylene-(6-10-membered aromatic ring), and -C₀₋₂ alkylene-(5-10-membered heteroaromatic ring);
R¹¹ and R¹² are each independently selected from the group consisting of hydrogen, halogen, -C₁₋₆ alkyl, halogen-substituted -C₁₋₆ alkyl, -C₀₋₂ alkylene-(3-10-membered cycloalkyl), -C₀₋₂ alkylene-(3-10-membered heterocycloalkyl), -C₀₋₂ alkylene-(6-10-membered aromatic ring), and -C₀₋₂ alkylene-(5-10-membered heteroaromatic ring);
R² is selected from the group consisting of hydrogen, halogen, cyano, nitro, -C₁₋₆ alkyl, halogen-substituted -C₁₋₆ alkyl, -C₀₋₂ alkylene-OR²¹, -C₀₋₂ alkylene-C(O)R²¹, -C₀₋₂ alkylene-C(O)NR²¹R²², -C₀₋₂ alkylene-NR²¹R²², -C₀₋₂ alkylene-NR²¹C(O)R²², -C₀₋₂ alkylene-(3-10-membered cycloalkyl), -C₀₋₂ alkylene-(3-10-membered heterocycloalkyl), -C₀₋₂ alkylene-(6-10-membered aromatic ring), and -C₀₋₂ alkylene-(5-10-membered heteroaromatic ring);
R²¹ and R²² are each independently selected from the group consisting of hydrogen, halogen, -C₁₋₆ alkyl, halogen-substituted -C₁₋₆ alkyl, -C₀₋₂ alkylene-(3-10-membered cycloalkyl), and -C₀₋₂ alkylene-(3-10-membered heterocycloalkyl);
R³ is selected from the group consisting of hydrogen, halogen, cyano, nitro, hydroxy, -C₁₋₆ alkyl, halogen-substituted -C₁₋₆ alkyl, -C₀₋₂ alkylene-OR³¹, -C₀₋₂ alkylene-C(O)R³¹, -C₀₋₂ alkylene-C(O)NR³¹R³², -C₀₋₂ alkylene-NR³¹R³², -C₀₋₂ alkylene-NR³¹C(O)R³², -C₀₋₂ alkylene-(3-10-membered cycloalkyl), -C₀₋₂ alkylene-(3-10-membered heterocycloalkyl), -C₀₋₂ alkylene-(6-10-membered aromatic ring), and -C₀₋₂ alkylene-(5-10-membered heteroaromatic ring);
R³¹ and R³² are each independently selected from the group consisting of hydrogen, halogen, -C₁₋₆ alkyl, halogen-substituted -C₁₋₆ alkyl, -C₀₋₂ alkylene-NR³³R³⁴, -C₀₋₂ alkylene-(3-10-membered cycloalkyl), -C₀₋₂ alkylene-(3-10-membered heterocycloalkyl), -C₀₋₂ alkylene-(6-10-membered aromatic ring), and -C₀₋₂ alkylene-(5-10-membered heteroaromatic ring);
R³³ and R³⁴ are each independently selected from the group consisting of hydrogen, halogen, -C₁₋₆ alkyl, and halogen-substituted -C₁₋₆ alkyl;
R⁴ is selected from the group consisting of hydrogen, -C₁₋₆ alkyl, and halogen-substituted -C₁₋₆ alkyl;
m is 0, 1 or 2; and n is 1 or 2;
wherein C₀ means that the radical group is absent.

In some embodiments of the present disclosure, R¹ is selected from the group consisting of hydrogen, -C₁₋₃ alkyl, -C₀₋₂ alkylene-NR¹¹R¹², and -(3-6-membered cycloalkyl).

In some embodiments of the present disclosure, R¹ is selected from the group consisting of hydrogen, methyl, cyclopropyl, and -NH₂.

In some embodiments of the present disclosure, R² is selected from the group consisting of halogen, -C₁₋₃ alkyl, and -C₀₋₂ alkylene-(3-10-membered cycloalkyl).

In some embodiments of the present disclosure, R² is selected from the group consisting of hydrogen, methyl, halogen, and cyclopropyl.

In some embodiments of the present disclosure, R³ is selected from the group consisting of -C₀₋₂ alkylene-OR³¹, -C₀₋₂ alkylene-NR³¹R³², -C₀₋₂ alkylene-NR³¹C(O)R³², and -(3-6-membered heterocycloalkyl);
R³¹ and R³² are each independently selected from the group consisting of hydrogen, -C₁₋₃ alkyl, and -C₀₋₂ alkylene-NR³³R³⁴;
R³³ and R³⁴ are each independently selected from the group consisting of hydrogen, and -C₁₋₃ alkyl.

In some embodiments of the present disclosure, R³ is selected from the group consisting of

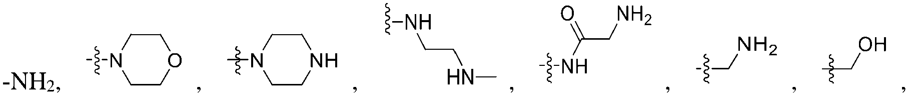

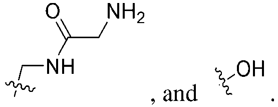

In some embodiments of the present disclosure, R⁴ is selected from the group consisting of hydrogen, and methyl.

In some embodiments of the present disclosure, R^{X1} and R^{X2} are each independently selected from the group consisting of hydrogen, and methyl.

In some embodiments of the present disclosure, R^{Y} is selected from the group consisting of hydrogen, methyl, and -C(O)R^{Y1}.

In some embodiments of the present disclosure, R^{Y1} is selected from the group consisting of hydrogen, and -C₁₋₃ alkyl.

In some embodiments of the present disclosure, the compound of Formula I is represented by Formula II: 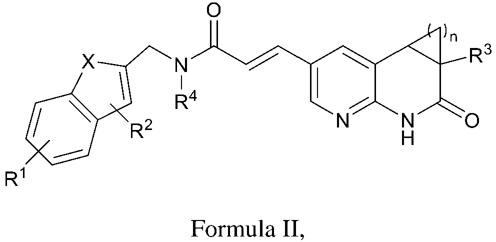 wherein R¹, R², R³, R⁴, X, and n are as defined previously.

In some embodiments of the present disclosure, the compound of Formula I is represented by Formula III: 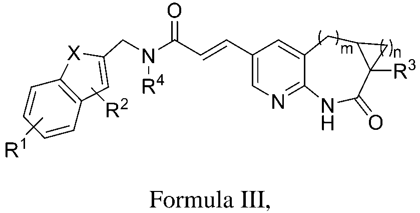 wherein R¹, R², R³, R⁴, X, m, and n are as defined previously.

In some embodiments of the present disclosure, the compound of Formula I is represented by Formula IV: 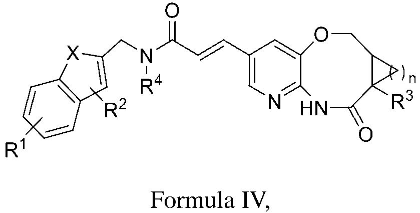 wherein R¹, R², R³, R⁴, X, and n are as defined previously.

In some embodiments of the present disclosure, the compound of Formula I is represented by Formula V: 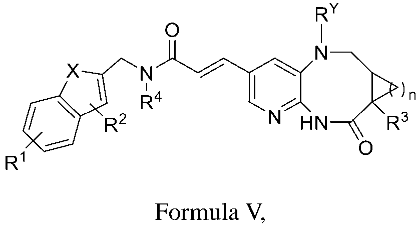 wherein R¹, R², R³, R⁴, R^{Y}, X, and n are as defined previously.

In some embodiments of the present disclosure, the compound of Formula I is selected from the group consisting of:

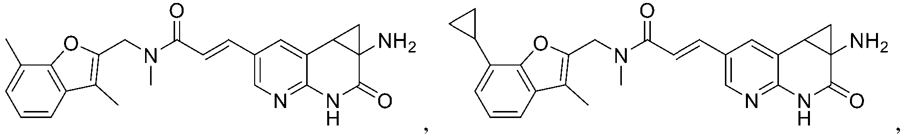

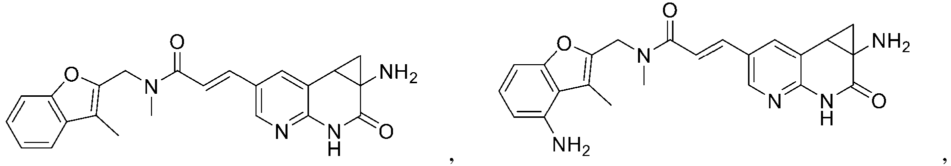

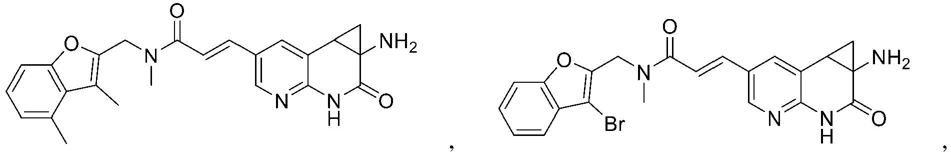

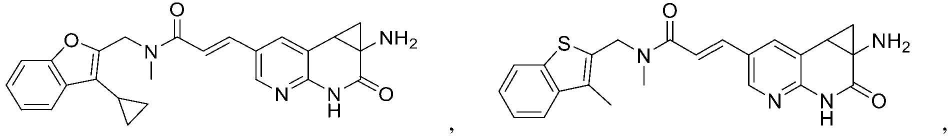

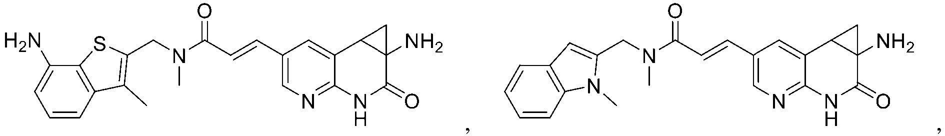

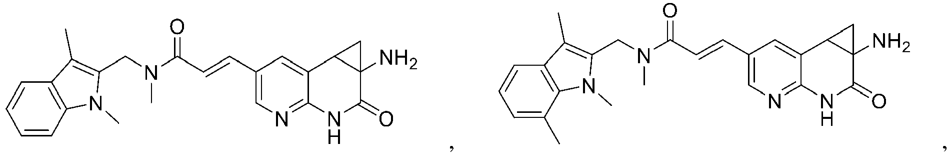

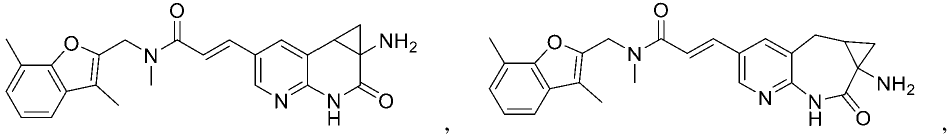

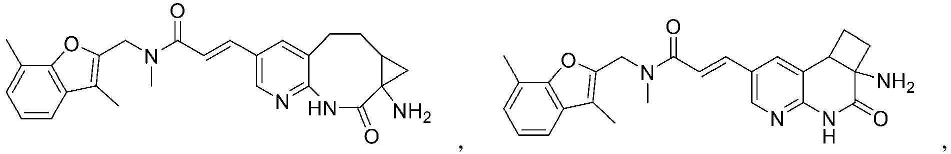

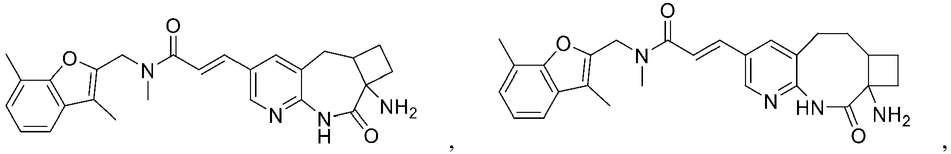

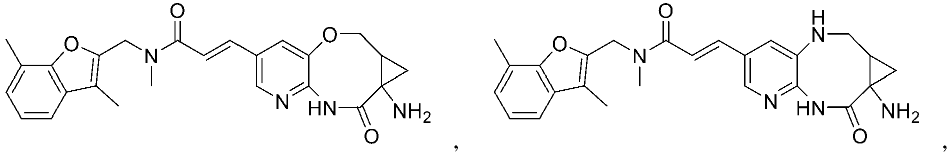

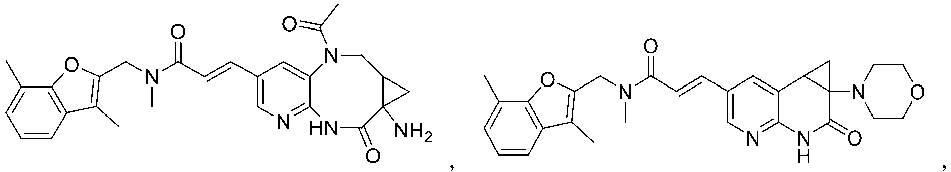

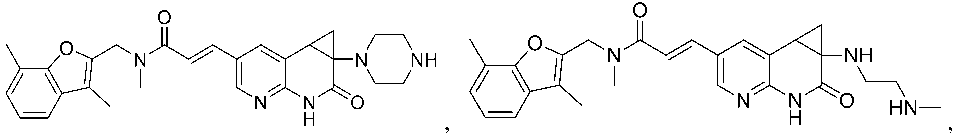

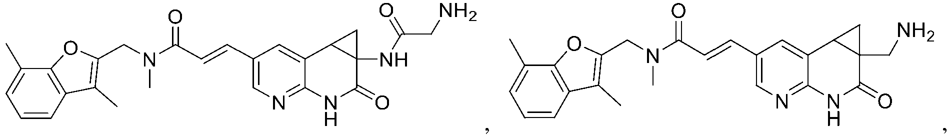

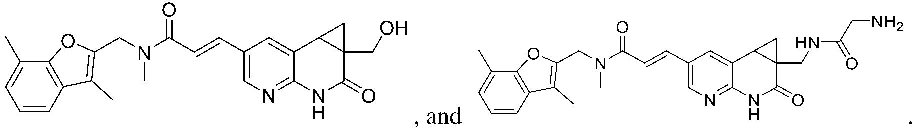

A second aspect of the present disclosure provides a pharmaceutical composition comprising the compound of Formula I, the stereoisomer, the deuterated compound, the isotopically labeled derivative, the pharmaceutically acceptable salt, the prodrug, or the solvate thereof.

In some embodiments of the present disclosure, the aforementioned pharmaceutical composition further includes a pharmaceutically acceptable carrier and/or an excipient and/or a vehicle.

The pharmaceutical composition of the present disclosure is suitable for various routes of administration and can therefore be formulated into any pharmaceutically acceptable dosage form. For example, the aforementioned pharmaceutical composition can be administered to a patient or a subject in need thereof by oral, parenteral, rectal or pulmonary administration, etc. For oral administration, the aforementioned pharmaceutical composition may be formulated as an oral formulation, for example a conventional oral solid formulation such as tablets, capsules, pills, granules, etc.; or an oral liquid solid formulation such as oral solutions, oral suspensions, syrups, etc. When formulated as an oral formulation, an appropriate filler, binder, disintegrant, lubricant, etc., may be added. For parenteral administration, the aforementioned pharmaceutical composition may also be formulated as an injection, including injection solutions, sterile powders for injection, and concentrated solutions for injection. When formulated as an injection, conventional methods in the existing pharmaceutical field can be used for production. When an injection is formulated, it is not necessary to add an additive, or an appropriate additive can be added according to the nature of a drug. For rectal administration, the aforementioned pharmaceutical composition may be formulated as a suppository, etc. For pulmonary administration, the aforementioned pharmaceutical composition may be formulated as inhalation preparations, aerosols, dry powder inhaler or sprays, etc.

The pharmaceutically acceptable excipient refers to a substance that is non-toxic, compatible with active ingredients, and biologically suitable for use in a living organism. The selection of a specific excipient will depend on the route of administration employed for treating a specific patient or type and condition of a disease. Examples of the pharmaceutically acceptable excipient include but are not limited to, solvents, diluents, dispersants, suspending agents, surfactants, isotonic agents, thickeners, emulsifiers, binders, lubricants, stabilizers, humectants, co-emulsifiers, buffers, absorbents, colorants, ion exchangers, release agents, coating agents, corrective agents, antioxidants, etc., conventionally used in the pharmaceutical field. When necessary, flavoring agents, preservatives, and sweeteners can also be added to the pharmaceutical composition.

A third aspect of the present disclosure provides use of the compound of Formula I, the stereoisomer, the deuterated compound, the isotopically labeled derivative, the pharmaceutically acceptable salt, the prodrug, or the solvate thereof, or the aforementioned pharmaceutical composition in the preparation of a drug for treating and/or preventing FabI enzyme-mediated diseases.

In some embodiments of the present disclosure, an effective FabI enzyme inhibitor that can be used for treating diseases caused by organisms requiring FabI as their main reductase is provided. The organisms include, but are not limited to, one or more of the following organisms: *Francisella tularensis, Staphylococcus aureus, Bacillus anthracis, Plasmodium falciparum, Yersinia pestis, Enterococcus faecalis, Staphylococcus epidermidis, Staphylococcus saprophyticus, Bordetella pertussis, Campylobacter jejuni, Brucella, Legionella pneumophila, Neisseria gonorrhoeae, Neisseria meningitidis, Rickettsia rickettsiae, Salmonella enterica, Salmonella, Vibrio cholerae, Chlamydia trachomatis, Chlamydia trachomatis pneumonia, Chlamydia pneumoniae, Chlamydia psittaci, Tuberculosis bacterium, Mycoplasma, Mycobacterium tuberculosis, Mycoplasma pneumoniae* and *Listeria monocytogenes.*

A fourth aspect of the present disclosure provides a method for treating and/or preventing FabI enzyme-mediated diseases, comprising administering an effective amount of the compound of Formula I, the stereoisomer, the deuterated compound, the isotopically labeled derivative, the pharmaceutically acceptable salt, the prodrug, or the solvate thereof, or the aforementioned pharmaceutical composition according to any of the above embodiments. The aforementioned diseases are as defined previously.

The method of the present disclosure can be a combination therapy that is combined with other therapies known in the art for treating specific diseases or indications. The aforementioned other therapies include the administration of other active drugs, which may be administered simultaneously, separately, or sequentially with the compounds or the pharmaceutical compositions containing the compounds of the present disclosure.

The aforementioned other therapies can also include physical or indirect therapies, such as surgical procedures, laser therapies, endocrine therapy (also known as hormone therapies), etc. Specifically, such therapies can be administered simultaneously with the administration of the compounds or the pharmaceutical compositions containing the compounds of the present disclosure, or may be administered at an interval before or after such administration.

The compounds and derivatives provided in the present disclosure can be named according to the International Union of Pure and Applied Chemistry (IUPAC) or Chemical Abstracts Service (CAS, Columbus, OH) nomenclature system.

Regarding the definition of terms used in the present disclosure, unless otherwise specified, the initial definitions provided for the groups or terms herein are suitable for the groups or terms throughout the entire specification; for terms that are not specifically defined herein, their meanings should be given by those skilled in the art based on the content and context of the disclosure.

The term "substitution" refers to the replacement of hydrogen atoms in a molecule with other different atoms or groups; or, the lone pair electrons of atoms in the molecule are replaced by other atoms or groups, for example the lone pair electrons on S atoms are replaced by O atoms to form

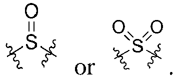

The phrase "may be further substituted" refers to the situation where "substitution" may occur but does not necessarily have to occur, and this phrase encompasses situations where substitution occurs or does not occur.

The term "multiple" refers to a quantity of two or more, and therefore the phrase "substituted by multiple groups" described in the present disclosure refers to being substituted by two or more groups. The actual number of the substituents is influenced by the number of substitutable sites and steric hindrance of the substituted groups. The term "multiple" generally refers to being substituted by two, three, four, five, or six groups, and further preferably by two or three groups.

The minimum and maximum carbon atom contents in the hydrocarbon are represented by its prefix, for example, the term C_{a-b} alkyl refers to any alkyl group containing "a" to "b" carbon atoms. Therefore, for example, C₁₋₆ alkyl refers to an alkyl group containing 1 to 6 carbon atoms.

The term "alkyl" refers to a saturated hydrocarbon chain with a specified number of member atoms. The alkyl group may be linear or branched. A representative branched alkyl group has one, two, or three branches. The alkyl group may be optionally substituted with one or more substituents as defined herein. The alkyl group includes methyl, ethyl, propyl (n-propyl and isopropyl), butyl (n-butyl, isobutyl, and tert-butyl), pentyl (n-pentyl, isopentyl, and neopentyl), and hexyl group. The alkyl group may also be a part of other groups, which are -O(C₁₋₆ alkyl), for example.

The term "alkylene" refers to a divalent saturated aliphatic hydrocarbon group with a specified number of member atoms. "C_{a-b} alkylene" refers to an alkylene group containing a to b carbon atoms. The alkylene group includes a branched and linear hydrocarbon group. For example, the term "propylene" may be exemplified by the following structure: 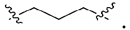 Similarly, the term "dimethylbutylene" may be exemplified by any of the following structures, for example:

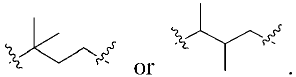

The -C₀₋₄ alkylene in the present disclosure may be C₀ alkylene, C₁ alkylene (e.g., -CH₂-), C₂ alkylene (e.g., -CH₂CH₂-, etc.), C₃ alkylene, or C₄ alkylene; C₀ alkylene refers to the absence of the group, which is connected in the form of a chemical bond; and A-C₀ alkylene-B refers to A-B, where groups A and B are directly connected via a chemical bond.

The term "alkenyl" refers to a linear or branched hydrocarbon group having at least one unsaturated ethenyl site (>C=C<). For example, C_{a-b} alkenyl refers to an alkenyl group having a to b carbon atoms and is intended to include, for example, ethenyl, propenyl, isopropenyl, 1,3-butadienyl, etc.

The term "alkynyl" refers to a linear or branched monovalent hydrocarbon containing at least one triple bond. The term "alkynyl" is also intended to include those hydrocarbon groups having one triple bond and one double bond. For example, C₂₋₆ alkynyl is intended to include ethynyl, propynyl, etc.

The term "cycloalkyl" as used herein refers to a saturated or partially saturated cyclic group having multiple carbon atoms, no cyclic heteroatoms, and a single ring or multiple rings (including fused rings). Examples of cycloalkyl groups include cyclopropyl, cyclobutyl, cyclohexyl, cyclopentyl, cyclooctyl, cyclopentenyl, cyclohexenyl, and polycyclic alkyl rings such as dicyclopropyl, dicyclopentyl, dicyclohexyl, dicycloocty, etc., where each ring in a polycyclic alkyl ring may be connected to the same carbon atom, such as 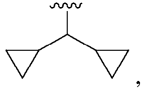 or it may be connected to adjacent and/or spaced different carbon atoms, such as

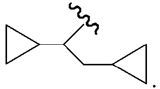

The term "heterocycloalkyl" or "heterocyclic" as used herein refers to a saturated ring or a non-aromatic partially saturated ring having a single ring or multiple rings (fused, bridged, spiro) containing at least one heteroatom, wherein the heteroatom refers to nitrogen, oxygen, sulfur, etc. It generally represents a monovalent saturated or partially unsaturated monocyclic or polycyclic ring system with multiple ring atoms, which contains 1, 2, or 3 ring heteroatoms selected from N, O, and S with the remaining ring atoms being carbon. Examples of heterocycloalkyl groups in a monocyclic heterocycloalkyl system include oxetanyl, azetidinyl, pyrrolidinyl, 2-oxo-pyrrolidin-3-yl, tetrahydrofuranyl, tetrahydrothiophenyl, pyrazolidinyl, imidazolidinyl, thiazolidinyl, piperidinyl, tetrahydropyranyl, tetrahydrothiopyranyl, piperazinyl, morpholinyl, thiomorpholinyl, 1,1-dioxo-thiomorpholin-4-yl, azepanyl, diazepanyl, homopiperazinyl, or oxazepanyl, etc. Examples of heterocycloalkyl groups in a fused heterocycloalkyl system include 8-azabicyclo[3.2.1]octyl, quinuclidinyl, 8-oxa-3-azabicyclo[3.2.1]octyl, 9-azabicyclo[3.3.1]nonyl, etc. Examples of heterocycloalkyl groups in a bridged heterocycloalkyl system include 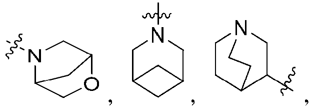 etc. Examples of heterocycloalkyl groups in a spiro heterocycloalkyl system include 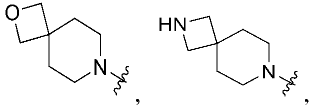 etc. Examples of partially saturated heterocycloalkyl groups include dihydrofuranyl, imidazolinyl, tetrahydropyridinyl, and dihydropyranyl, etc. The term "heterocycloalkyl" also includes a partially saturated cyclic group formed by fusion of an aromatic ring containing at least one heteroatom with a non-aromatic ring, and the point of attachment may be located at a non-aromatic carbon atom, an aromatic carbon atom, or a heteroatom, examples of which include

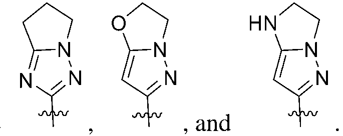

The term "aromatic ring" as used herein refers to an aromatic hydrocarbon group having multiple carbon atoms. The aryl is generally a monocyclic, bicyclic, or tricyclic aromatic group having multiple carbon atoms. In addition, the term "aryl" as used herein refers to an aromatic substituent that may be a single aromatic ring or multiple aromatic rings fused together. Non-limiting examples include phenyl, naphthyl, and tetrahydronaphthyl.

The term "heteroaromatic ring" as used herein refers to an aromatic unsaturated ring containing at least one heteroatom, wherein the heteroatom refers to nitrogen, oxygen, sulfur, etc. It generally includes aromatic monocyclic or bicyclic hydrocarbons containing multiple ring atoms, with one or more ring atoms selected from O, N, and S. Preferably, there are one to three heteroatoms. Examples of heteroaryl include pyridinyl, indolyl, quinoxalinyl, quinolinyl, isoquinolinyl, benzothienyl, benzofuranyl, benzothienyl, benzopyranyl, benzothiopyranyl, furanyl, pyrrolyl, thiazolyl, oxazolyl, isoxazolyl, triazolyl, tetrazolyl, pyrazolyl, imidazolyl, thienyl, oxadiazolyl, benzimidazolyl, benzothiazolyl, and benzoxazolyl.

The term "halogen" as used herein refers to fluorine, chlorine, bromine, or iodine.

The phrase "halogen-substituted alkyl" as used herein means that one or more hydrogen atoms in the alkyl are substituted by halogen. For example, halogen-substituted C₁₋₄ alkyl refer to an alkyl containing 1 to 4 carbon atoms in which one or more hydrogen atoms are substituted by one or more halogen atoms; examples include monofluoromethyl, difluoromethyl, and trifluoromethyl.

The terms "-OR", "-NR", "-NRR", etc., as used herein means that the R group is bonded to an oxygen atom or a nitrogen atom via a single bond.

The terms "-C(O)R", "-S(O)₂R", etc., as used herein means that the oxygen atom is bonded to a carbon or a sulfur atom via a double bond.

The terms "-C(O)R", "-S(O)₂R", etc., as used herein means that the oxygen atom is bonded to a carbon or a sulfur atom via a double bond, and the R group is bonded to an oxygen or a sulfur atom via a single bond. For example, "-S(O)(NH)R" means that the oxygen atom is bonded to the sulfur atom via a double bond and the nitrogen atom is bonded to the sulfur atom via a double bond, and the R group is bonded to the sulfur atom via a single bond.

The "oxo" as used herein refers to =O, which means that the oxygen atom replaces two hydrogen atoms or a lone pair of electrons through a double bond.

The "-", "---", and " 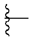 " in the description of a group of the present disclosure are used to describe the position where the group is substituted. For example, 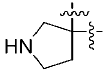 means that the tetrahydropyrrole ring forms a spiro ring with other rings in the structure at the position of " 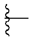 ".

The term "deuterated compound" as used herein refers to a molecule or group in which one or more hydrogen atoms are substituted by deuterium atoms, with the abundance of deuterium being greater than its natural abundance.

The term "stereoisomer" includes enantiomers and diastereomers, as well as cis-trans isomers, tautomers, etc.

The phrase "pharmaceutically acceptable" means that a carrier, a vehicle, a diluent, an excipient, and/or a formed salt is typically chemically and physically compatible with other components of a pharmaceutical dosage form and physiologically compatible with a recipient.

The terms "salt" and "pharmaceutically acceptable salt" refer to an acid addition salt and/or base addition salt formed by the aforementioned compounds or stereoisomers thereof with an inorganic and/or an organic acid and base, as well as a zwitterionic salt (an inner salt) and a quaternary ammonium salt such as an alkylammonium salt. These salts may be directly obtained during the final separation and purification of a compound. It may also be obtained by mixing the aforementioned compounds or stereoisomers thereof with a certain amount of acid or base appropriately (e.g., in an equivalent amount). These salts may form precipitates in solution and be collected by filtration, or recovered after evaporation of the solvent, or prepared by lyophilization after reaction in an aqueous medium.

The term "prodrug" as used herein refers to a compound that rapidly converts into the parent compound of the above formula in vivo, and which can be converted into the compounds of the present disclosure through chemical or biochemical methods in either in vivo or in vitro environments, for example, by hydrolysis in the blood.

The compounds of the present disclosure may exist in both non-solvated and solvated forms, and the solvated form includes a hydrate form. Generally, the solvated form is equivalent to the non-solvated form and is also encompassed within the scope of the present disclosure.

In some embodiments, one or more compounds of the present disclosure may be used in combination with each other. Alternatively, the compounds of the present disclosure may be used in combination with any other active agents for the preparation of a drug or pharmaceutical composition for regulating cellular function or treating diseases. If a combination of compounds is used, these compounds may be administered to a subject simultaneously, separately, or sequentially.

In the present disclosure, when any variable appears more than once in the composition or structure of a compound, its definition in each case is independent, and the substituents may be the same or different. For example, when a group is substituted by multiple R¹¹, each R¹¹ is independent.

Obviously, based on the above contents of the present disclosure, various other forms of modifications, substitutions, or changes may be made in accordance with common technical knowledge and customary means in the art without departing from the basic technical ideas of the present disclosure.

The present disclosure has the following beneficial effects. The FabI enzyme inhibitor of the present disclosure has superior drugability compared to the existing FabI inhibitors under clinical investigations, and may be directly administered in the form of the parent drug, resulting in enhanced in vivo exposure. In terms of antibacterial spectrum, compared to the existing FabI inhibitors under clinical investigations, the compound in the present disclosure demonstrates a broader spectrum, including activity against the common pathogen of community-acquired infections, Moraxella catarrhalis. In terms of in vivo drug efficacy, the compounds of the present disclosure exhibit superior in vivo drug efficacy compared to the existing FabI inhibitors under clinical investigations. In summary, the present disclosure provides a new option for clinical screening and/or preparation of drugs for the treatment of diseases associated with FabI enzyme activity.

### BRIEF DESCRIPTION OF DRAWINGS

FIG. 1 shows pharmacokinetic test results of compound 1a of the present disclosure in rats.
FIG. 2 shows the pharmacokinetic test results of Debio-1450 in rats.
FIG. 3 shows the in vivo efficacy test results of the compounds of the present disclosure in mice.

### DETAILED DESCRIPTION

The content of the present disclosure will be further described in detail below through specific embodiments. Unless otherwise specified, the raw materials, reagents, or devices used in the examples and comparative examples may be obtained from conventional commercial channels or through existing technical methods. Unless otherwise specified, the experimental or testing methods are conventional methods in the art.

The structures of the compounds were determined by nuclear magnetic resonance (NMR) and mass spectrometry (MS). The NMR chemical shifts (δ) were reported in parts per million (ppm, 10⁻⁶). NMR spectra were recorded on Bruker Avance III 400 and Bruker Avance 300 spectrometers, with deuterated dimethyl sulfoxide (DMSO-d₆), deuterated chloroform (CDCl₃), or deuterated methanol (CD₃OD) as solvents, and tetramethylsilane (TMS) as an internal standard.

LC-MS analysis was performed using a Liquid Chromatography-Mass Spectrometer (Shimadzu LC-MS 2020 (ESI)). HPLC analysis was performed using Shimadzu high-performance liquid chromatograph (Shimadzu LC-20A). Preparative medium-pressure liquid chromatography (MPLC) was performed on Gilson GX-281 reversed-phase preparative chromatograph. Thin-layer chromatography (TLC) was performed using Yantai Huanghai HSGF254 or Qingdao GF254 silica gel plates. The thickness of the plates for separation and purification of products was 0.4 mm to 0.5 mm. Column chromatography was generally performed using Yantai Huanghai silica gel (200-300 mesh).

The known starting materials in the present disclosure may be synthesized according to methods known in the art, or may be purchased from companies such as Energy Chemical, Chengdu Kelong Chemical, Accela ChemBio Co. Ltd, and J&K Scientific, etc in China.

Unless otherwise specified in the Examples, the reactions are carried out under a nitrogen atmosphere. Unless otherwise specified in the Examples, the solution refers to an aqueous solution. Unless otherwise specified in the Examples, the reaction temperature is room temperature. Unless otherwise specified in the Examples, M refers to moles per liter.

### Example 1

In this example, a compound **1a/1b** (chemical name: (*E*)-3-(1a-amino-2-oxa-1a,2,3,7b-tetrahydro-1H-cyclopropa[c][1,8]naphthyridin-6-yl)-N-((3,7 -dimethylbenzofuran-2-yl)methyl)-N-methylacrylamide) was prepared. The specific process was follows:

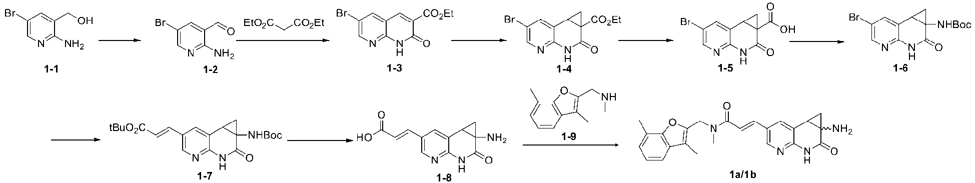

### Step 1

Compound **1-1** (6.0 g, 29.5 mmol) was dissolved in dichloromethane (200.0 mL), and then active manganese dioxide (25.7 g, 295.0 mmol) was added at room temperature to obtain a reaction mixture. The reaction mixture was stirred at 40°C for 3 hours. Upon completion of the reaction as monitored by TLC, the resulting reaction product was filtered, and the resulting filtrate was concentrated under reduced pressure to remove solvent to afford crude product **1-2** (5.0 g, 24.9 mmol, 84.3% yield), which was directly used for the next step.

MS-ESI calcd for [M+H]⁺: 202.0; found: 202.0.

### Step 2

Compound **1-2** (5.0 g, 24.9 mmol), diethyl malonate (39.8 g, 249.0 mmol), and piperidine (4.2 g, 49.7 mmol) were mixed and reacted at 120°C for 2 hours. The reaction was monitored by LCMS. Upon completion, the resulting reaction product was filtered, and a filter cake was washed with ethyl acetate (3×20 mL) to afford crude product **1-3** (5.8 g, 19.5 mmol, 78.4% yield), which was directly used for the next step.

MS-ESI calcd for [M+H]⁺: 298.0; found: 298.0.

### Step 3

Trimethylsulfoxonium iodide (8.0 g, 40.0 mmol) was dissolved in dimethyl sulfoxide (150.0 mL), and then sodium hydride (2.4 g, 60.0 mmol) was added. The resulting mixture was reacted at 25 ° C under the protection of nitrogen for 1 hour with stirring, and then compound **1-3** (5.8 g, 19.5 mmol) was added. The reaction was continued for 3 hours. After the starting material was completely consumed, the reaction solution was poured into a saturated aqueous ammonium chloride solution (500.0 mL) to quench the reaction. The resulting reaction product was extracted with ethyl acetate (3 ×200.0 mL). The obtained organic phase was washed with saturated brine, dried over anhydrous sodium sulfate, and filtered, and the resulting filtrate was concentrated under reduced pressure to remove solvent to afford crude product **1-4** (4.5 g, 14.5 mmol, 74.2% yield), which was directly used for the next step.

MS-ESI calcd for [M+H]⁺: 312.0; found: 312.0.

### Step 4

Compound **1-4** (4.5 g, 14.5 mmol) was dissolved in a mixture of tetrahydrofuran (60.0 mL) and water (60.0 mL), and then lithium hydroxide (693.5 mg, 28.9 mmol) was added. The resulting mixture was stirred at room temperature for 2 hours. Upon completion of reaction, an appropriate amount of dilute hydrochloric acid was added to adjust the pH below 6. The resulting reaction product was extracted with ethyl acetate (3 ×100.0 mL). The obtained organic phase was dried over anhydrous sodium sulfate, and filtered, and the resulting filtrate was concentrated under reduced pressure to remove solvent to afford crude product **1-5** (2.9 g, 10.2 mmol, 70.4% yield), which was directly used for the next step.

MS-ESI calcd for [M+H]⁺: 284.0; found: 284.0.

### Step 5

Compound **1-5** (2.9 g, 10.2 mmol) was added to toluene (45.0 mL), then diphenylphosphoryl azide (4.2 g, 15.4 mmol), triethylamine (3.1 g, 30.7 mmol), and tert-butanol (1.1 g, 15.4 mmol) were added sequentially. The resulting mixture was reacted at 100°C for 4 hours under stirring. Upon completion of reaction, toluene was removed by concentration under reduced pressure, and a resulting crude product was purified by liquid chromatography to afford compound **1-6** (1.5 g, 4.2 mmol, yield: 41.5%).

MS-ESI calcd for [M+H]⁺: 355.0; found: 355.0.

### Step 6

Compound **1-6** (1.5 g, 4.2 mmol) was dissolved in *N,N*-dimethylformamide (40.0 mL). Then, *tert*-butyl acrylate (1.1 g, 8.5 mmol), tris(dibenzylideneacetone)dipalladium (388.0 mg, 0.4 mmol), tri(*o*-tolyl)phosphine (257.7 mg, 0.85 mmol), and *N,N*-diisopropylethylamine (1.1 g, 8.5 mmol) were added at room temperature. The resulting mixture was reacted at 100°C for 3 hours. Upon completion, the reaction was quenched with water (30 mL). The resulting reaction product was extracted with ethyl acetate (3 ×50.0 mL). The obtained organic phase was washed with saturated brine, dried over anhydrous sodium sulfate, and filtered, and the resulting filtrate was concentrated under reduced pressure to remove solvent to afford a crude product. The crude product was purified by column chromatography to afford compound **1-7** (1.0 g, 2.5 mmol, 58.8% yield).

MS-ESI calcd for [M+H]⁺: 402.2; found: 402.2.

### Step 7

Compound **1-7** (1.0 g, 2.5 mmol) was dissolved in dichloromethane (30 mL), and trifluoroacetic acid (10 mL) was added. Then, the resulting mixture was stirred at room temperature for 1 hour. Upon completion of reaction, the resulting reaction product was filtered and concentrated under reduced pressure to remove solvent to afford crude product **1-8** (2.0 g).

MS-ESI calcd for [M+H]⁺: 246.1; found: 246.1.

### Step 8

Crude product **1-8** (300.0 mg) was dissolved in *N,N*-dimethylformamide (3.0 mL), then compound **1-9** (70.0 mg, 0.4 mmol), 2-(7-azabenzotriazol-1-yl)-*N,N,N',N'*-tetramethyluronium hexafluorophosphate (154.6 mg, 0.4 mmol), and *N,N*-diisopropylethylamine (95.0 mg, 0.7 mmol) were added sequentially at room temperature. The resulting mixture was reacted at 25°C for 1 hour under stirring. Upon completion, the reaction was quenched with water (2 mL). The resulting reaction product was extracted with ethyl acetate (3×10.0 mL). The obtained organic phase was washed with saturated brine, dried over anhydrous sodium sulfate, and filtered, and the resulting filtrate was concentrated under reduced pressure to remove solvent to afford a crude product. The crude product was purified by liquid chromatography and then separated by supercritical fluid chromatography (SFC) to afford compound **1a** (30.0 mg, 0.07 mmol) with a purity of 100.0% and compound **1b** (26.5 mg, 0.06 mmol) with a purity of 100.0%.

SFC separation conditions: Column specification: 3 µm, 150 mm ×3 mm.

Mobile Phrase: A: CO₂, B: EtOH/0.1%DEA, Flow Rate: 1 mL/min Column Temperature: 40°C. [P1, 2.834 min; P2, 3.718 min].
**1a:** MS-ESI calcd for [M+H]⁺: 417.2; found: 417.2.
**1b:** MS-ESI calcd for [M+H]⁺: 417.2; found: 417.2.
**1a**: 1H NMR (400 MHz, DMSO) δ 8.34 (d, J = 12.9 Hz, 1H), 8.26 (s, 1H), 7.49 (dd, J = 14.6, 12.0 Hz, 1H), 7.38 (d, J = 7.4 Hz, 1H), 7.22 (dd, J = 25.2, 9.8 Hz, 1H), 7.17-7.05 (m, 2H), 4.84 (t, J = 42.4 Hz, 2H), 3.27-2.96 (m, 3H), 2.59 (dt, J = 16.2, 8.1 Hz, 1H), 2.47-2.33 (m, 3H), 2.24 (t, J = 7.3 Hz, 3H), 1.68-1.58 (m, 1H), 0.75 (d, J = 4.6 Hz, 1H).
**1b**: 1H NMR (400 MHz, DMSO) δ 8.37 (d, J = 12.2 Hz, 1H), 8.29 (s, 1H), 7.62-7.44 (m, 1H), 7.38 (d, J = 7.4 Hz, 1H), 7.23 (d, J = 15.4 Hz, 1H), 7.18-7.02 (m, 2H), 4.89 (d, J = 73.8 Hz, 2H), 3.28-2.94 (m, 3H), 2.71 (dd, J = 9.4, 5.7 Hz, 1H), 2.38 (d, J = 36.2 Hz, 3H), 2.24 (t, J = 7.4 Hz, 3H), 1.71 (d, J = 5.1 Hz, 1H), 0.87 (d, J = 4.1 Hz, 1H).

### Example 2

In this example, a compound **2a/2b** (chemical name: (*E*)-3-(1a-amino-2-oxa-1a,2,3,7b-tetrahydro-1*H*-cyclopropa[c][1,8]naphthyridin-6-yl)-N-((7-c yclopropyl-3-methylbenzofuran-2-yl)methyl)-N-methylacrylamide) was prepared. The specific process was as follows:

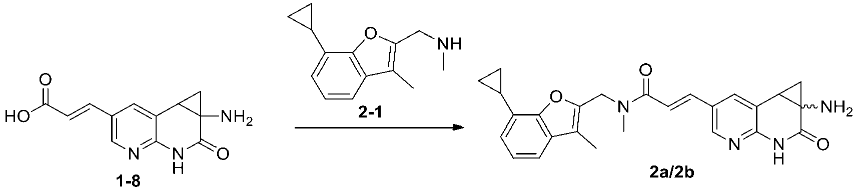

Crude product **1-8** (300.0 mg) was dissolved in *N,N*-dimethylformamide (3.0 mL), and then compound **2-1** (80.0 mg, 0.4 mmol), 2-(7-azabenzotriazol-1-yl)-*N,N,N',N'*-tetramethyluronium hexafluorophosphate (154.6 mg, 0.4 mmol), and *N,N*-diisopropylethylamine (95.0 mg, 0.7 mmol) were added sequentially at room temperature. The resulting mixture was reacted at 25°C for 1 hour under stirring. Upon completion, the reaction was quenched with water (2 mL). The resulting reaction product was extracted with ethyl acetate (10.0 mL×3). The obtained organic phase was washed with saturated brine, dried over anhydrous sodium sulfate, and filtered, and the resulting filtrate was concentrated under reduced pressure to remove solvent to afford a crude compound. The crude compound was purified by liquid chromatography and then separated by SFC to obtain compound **2a** (35.0 mg, 0.08 mmol) with a purity of 96.0% and compound **2b** (30.5 mg, 0.07 mmol) with a purity of 96.0%. SFC separation conditions: Column specification: 3 µm, 150 mm×3 mm.

Mobile Phase: A: CO₂, B: EtOH/0.1%DEA, Flow Rate: 1 mL/min Column Temperature: 40°C. [P1, 3.897 min; P2, 4.780 min].
**2a:** MS-ESI calcd for [M+H]⁺: 443.2; found: 443.2.
**2b:** MS-ESI calcd for [M+H]⁺: 443.2; found: 443.2.
**2a:** 1H NMR (400 MHz, DMSO) δ 8.34 (d, J = 11.0 Hz, 1H), 8.27 (d, J = 8.1 Hz, 1H), 7.52 (dt, J = 15.3, 11.5 Hz, 1H), 7.33 (d, J = 6.9 Hz, 1H), 7.22 (d, J = 15.4 Hz, 1H), 7.15 (td, J = 7.5, 3.9 Hz, 1H), 6.90 (dd, J = 16.6, 7.4 Hz, 1H), 4.92 (dd, J = 52.0, 33.8 Hz, 2H), 3.12 (d, J = 102.5 Hz, 3H), 2.66 (s, 1H), 2.25 (d, J = 7.3 Hz, 3H), 2.11 (d, J = 73.1 Hz, 1H), 1.68 (d, J = 4.6 Hz, 1H), 1.03 (d, J = 6.4 Hz, 1H), 0.89 (s, 2H), 0.79 (dd, J = 14.0, 8.7 Hz, 2H).
**2b:** 1H NMR (400 MHz, DMSO) δ 8.43 (d, J = 12.3 Hz, 1H), 8.36 (d, J = 9.8 Hz, 1H), 7.55 (dt, J = 15.1, 9.9 Hz, 1H), 7.33 (d, J = 7.6 Hz, 1H), 7.27 (t, J = 12.5 Hz, 1H), 7.15 (td, J = 7.6, 3.1 Hz, 1H), 6.91 (dd, J = 19.2, 7.4 Hz, 1H), 4.88 (d, J = 69.2 Hz, 2H), 3.29-2.98 (m, 3H), 2.94 (s, 1H), 2.25 (d, J = 6.2 Hz, 3H), 2.22-1.98 (m, 1H), 1.97-1.86 (m, 1H), 1.03 (d, J = 6.3 Hz, 1H), 0.87 (dd, J = 19.3, 5.3 Hz, 2H), 0.83-0.72 (m, 2H).

### Example 3

In this example, a compound **3a/3b** (chemical name: (*E*)-3-(1a-amino-2-oxa-1a,2,3,7b-tetrahydro-1*H*-cyclopropa[c][1,8]naphthyridin-6-yl)-N-((3-methylbenzofuran-2-yl)methyl)-N-methylacrylamide) was prepared. The specific process was as follows:

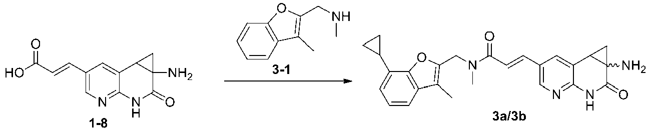

Crude product **1-8** (300.0 mg) was dissolved in N,N-dimethylformamide (3.0 mL), and then compound **3-1** (65.0 mg, 0.4 mmol), 2-(7-azabenzotriazol-1-yl)-*N,N,N',N'*-tetramethyluronium hexafluorophosphate (154.6 mg, 0.4 mmol), and *N,N*-diisopropylethylamine (95.0 mg, 0.7 mmol) were added sequentially at room temperature. The resulting mixture was reacted at 25°C for 1 hour under stirring. Upon completion, the reaction was quenched with water (2 mL). The resulting reaction product was extracted with ethyl acetate (10.0 mL×3). The obtained organic phase was washed with saturated brine, dried over anhydrous sodium sulfate, and filtered, and the resulting filtrate was concentrated under reduced pressure to remove solvent to afford a crude product. The crude product was purified by liquid chromatography and then separated by SFC to obtain compound **3a** (25.0 mg, 0.06 mmol) with a purity of 97.0% and compound **3b** (22.0 mg, 0.05 mmol) with a purity of 100.0%.

SFC separation conditions: Column specification: 3 µm, 150 mm×3 mm.

Mobile Phase: A: CO₂, B: EtOH/0.1%DEA, Flow Rate: 1 mL/min Column Temperature: 40°C. [P1, 3.541 min; P2, 5.085 min].
**3a:** MS-ESI calcd for [M+H]⁺: 403.2; found: 403.2.
**3b:** MS-ESI calcd for [M+H]⁺: 403.2; found: 403.2.
**3a:** 1H NMR (400 MHz, DMSO) δ 8.35 (t, J = 12.7 Hz, 1H), 8.28 (s, 1H), 7.57 (t, J = 9.3 Hz, 1H), 7.55-7.43 (m, 2H), 7.37-7.18 (m, 3H), 4.89 (d, J = 75.0 Hz, 2H), 3.24-2.93 (m, 3H), 2.73 (dd, J = 9.2, 5.7 Hz, 1H), 2.27 (s, 3H), 1.73 (s, 1H), 0.86 (d, J = 4.4 Hz, 1H).
**3b:** 1H NMR (400 MHz, DMSO) δ 8.36-8.29 (m, 1H), 8.26 (s, 1H), 7.57 (dd, J = 13.0, 11.8 Hz, 1H), 7.53-7.44 (m, 2H), 7.35-7.17 (m, 3H), 4.84 (dd, J = 53.0, 37.4 Hz, 2H), 3.23-2.94 (m, 3H), 2.65 (dd, J = 9.3, 5.6 Hz, 1H), 2.27 (s, 3H), 1.72-1.61 (m, 1H), 0.76 (d, J = 4.8 Hz, 1H).

### Test Example 1

In this test example, the enzymatic activity was evaluated by monitoring the oxidation of cofactor NADH/NADPH using a microplate reader.

The compound was dissolved in DMSO and added to a 384-well plate using ECHO665. Serial 3-fold or 4-fold dilutions were prepared to generate 10 dose points with two replicates per concentration, ensuring a final DMSO concentration 1% in each reaction well. Then, a FabI protein solution was added, and the protein was prepared in a buffer containing 100 mmol/L PBS, 0.05% Tween-20, and 150 mmol/L NaCl (pH 6.5), so that the final concentrations of SaFabI and EcFabI proteins were 15 nmol/L and 9 nmol/L, respectively. The prepared 5 µL of 15 nmol/L SaFabI and 9 nmol/L EcFabI were added to a 384-well assay plate, and centrifuged at 1000 rpm for 1 minute. Then, the plate was pre-incubated in a temperature-controlled shaker at 30 °C for 20 minutes. Moreover, substrates trans-octenoyl-CoA, NADPH, trans-dodecenoyl-CoA, and NADH were diluted in a buffer containing 100 mmol/L PBS, 0.05% Tween-20, and 15 0 mmol/L NaCl (pH 6.5) respectively, and their concentrations were adjusted to 300 µmol/L, 300 µmol/L, 600 µmol/L, and 150 µmol/L, respectively. For SaFabI, 5 µL of 300 µmol/L trans-octenoyl-CoA and 5 µL of 300 µmol/L NADPH were added to initiate the reaction. Immediately after initiation, the plate was placed into the microplate reader, and fluorescence signals (Ex 340 nm/Em 460 nm) were recorded every 0.5 minutes for at least 10 minutes. Similarly, 5 µL of 600 µmol/L trans-dodecenoyl-CoA and 5 µL of 150 µmol/L NADH were added to initiate reaction of EcFabI. Immediately after initiation, the plate was immediately placed into the microplate reader, and fluorescence signals (Ex 340 nm/Em 460 nm) were recorded every 0.5 minutes for at least 10 minutes. The reaction rate (fluorescence signal per minute) of each well was calculated from the fluorescence values. The inhibitory effect of the compound on the protein was then calculated based on the reaction rate, and the IC₅₀ value for inhibition of enzyme activity by the compound was determined by fitting the data using GraphPad Prism 6 with log (inhibitor) vs. response variable slope model. The fitting equation is: Y=Bottom + (Top-Bottom)/(1+10^((LogIC₅₀-X)*HillSlope)), wherein Y represents the known percentage of residual enzyme activity, and X represents the known Log concentration of the compound.

The in vitro enzymatic activity of the compounds in the present disclosure was shown through the above experiments. It is shown that the compounds in the present disclosure have excellent inhibitory activity against FabI enzymes in *Staphylococcus aureus* and *Escherichia coli.*

**Table 1 Evaluation of SaFabI&EcFabI Enzyme Activity**

| **Compound Nos.** | ***Sa*FabI IC₅₀ (µmol/L)** | ***Ec*FabI IC₅₀ (µmol/L)** |
|---|---|---|
| **Debio-1452** | 0.002 | 0.003 |
| **1a** | 0.005 | 0.004 |
| **1b** | 0.012 | 0.005 |
| **2a** | 0.010 | 0.006 |
| **2b** | 0.012 | 0.013 |
| **3a** | 0.010 | 0.007 |
| **3b** | 0.010 | 0.007 |

### Test Example 2

In this test example, the minimum inhibitory concentration (MIC) of the compound was determined.

The test drug concentration was set to be within a range of 0.008 µg/mL to 128 µg/mL in a two-fold serial dilution using an agar plate two-fold dilution method. The sample was dissolved in DMSO to prepare a 2 mL solution with a concentration of 1.92 mg/mL. The control group was prepared in sterile distilled water to obtain a concentration of 1.92 mg/mL. 1 mL of the prepared drug solution was added to a sterile culture dish, followed by addition of 14 mL of MHA culture medium at approximately 60°C. After thorough mixing, 1 mL of sterilized distilled water was added for two-fold dilution, resulting in a final drug concentration of 64 µg/mL, 32 µg/mL, 16 µg/mL, 8 µg/mL, 4 µg/mL, 2 µg/mL, 1 µg/mL, 0.5 µg/mL, 0.25 µg/mL, 0.125 µg/mL, 0.06 µg/mL, 0.03 µg/mL, 0.015 µg/mL, and 0.008 µg/mL (wherein the final concentrations of samples 2, 4, and 9 were diluted downward starting from 128 µg/mL), and set aside for later use. The prepared bacterial suspension was inoculated onto drug-containing MHA culture medium with a multi-point inoculator, with an inoculation volume of approximately 10⁴ CFU per point. A sterile control group (1 mL DMSO + 14 mL MHA culture medium and 1 mL sterile distilled water + 14 mL MHA culture medium) was set up. The plates were incubated at 37°C for 16-20 hours, and bacterial growth at each inoculation point was observed to determine its MIC value.

The above experiments have shown that the compounds of the present disclosure exhibit in vitro antibacterial activity, and excellent antibacterial activity against *Staphylococcus aureus,* including methicillin-sensitive *Staphylococcus aureus* (MSSA), methicillin-resistant *Staphylococcus aureus* (MRSA), and vancomycin-resistant *Staphylococcus aureus* (VRSA), and also exhibit moderate antibacterial activity against *Escherichia coli,* and weak antibacterial activity against *Klebsiella pneumoniae* and *Acinetobacter baumannii.*

**Table 2 Minimum Inhibitory Concentration MIC (µg/mL)**

| **Microbial Strains** | **MSSA** | **VRSA** | **MRSA** | ***Escherichia coli.*** | ***Klebsiella pneumoniae*** | ***Acinetobacter baumannii*** |
|---|---|---|---|---|---|---|
| **Compound Nos.** | **ATCC 29213** | **VRS 1** | **NRS 384** | **ATCC 25923** | **ATCC BAA 2472** | **ARLG 1852** |
| **Debio-1452** | <0.008 | 0.12 | <0.008 | 4 | >8 | >8 |
| **1a** | <0.008 | 0.25 | <0.008 | 4 | 16 | >32 |
| **1b** | <0.008 | 0.12 | <0.008 | 4 | 16 | >32 |
| 2a | <0.008 | 0.06 | <0.008 | 8 | 32 | >32 |
| **2b** | 0.016 | 0.06 | <0.008 | >16 | 32 | >32 |
| **3a** | 0.03 | 0.5 | 0.03 | 8 | >32 | >32 |
| **3b** | 0.016 | 0.25 | 0.016 | 8 | >32 | >32 |

Referring to the minimum inhibitory concentration test method mentioned above, the antibacterial spectrum of compound 1b was tested. The test results show that compound 1b exhibits excellent antibacterial activity against Staphylococcus species, such as *Staphylococcus epidermidis*, *Staphylococcus hominis*, *Staphylococcus hominis* , as well as *Moraxella catarrhalis.* The compound 1b also exhibits a good antibacterial activity against *Haemophilus influenzae.*

**Table 3 Antibacterial Spectrum Testing of Compound 1b MIC (µg/mL)**

| **Microbi al Strains** | ***Neisseria gonorrhoeae*** | ***Enterobact er cloacae*** | ***Haemophilus influenzae*** | ***Acinetobacter baumannii*** | ***Salmonella* Typhi** | ***Klebsiella pneumoniae*** |
|---|---|---|---|---|---|---|
| **Strain Nos.** | **ATCC49226** | **ATCC1304 7** | **ATCC29213** | **ATCC17979** | **CMCC12055** | **ATCC2055** |
| **MIC** | 8 | 8 | 0.25 | 32 | 4 | 8 |

| **Microbi al Strains** | ***Pseudomon as aeruginosa*** | ***Moraxella catarrhalis*** | ***Staphylococc us epidermidis*** | ***Staphylococc us haemolyticus*** | ***Staphylococc us hominis*** | ***Enterococc us faecalis*** |
|---|---|---|---|---|---|---|
| **Strain Nos.** | **ATCC12055** | **ATCC4361 7** | **ATCC35983** | **CICC23976** | **ATCC27844** | **ATCC5129 9** |
| **MIC** | >64 | <0.06 | <0.06 | <0.06 | <0.06 | >64 |

### Test Example 3

In this test experiment, a pharmacokinetic study of the compound in rats was conducted.

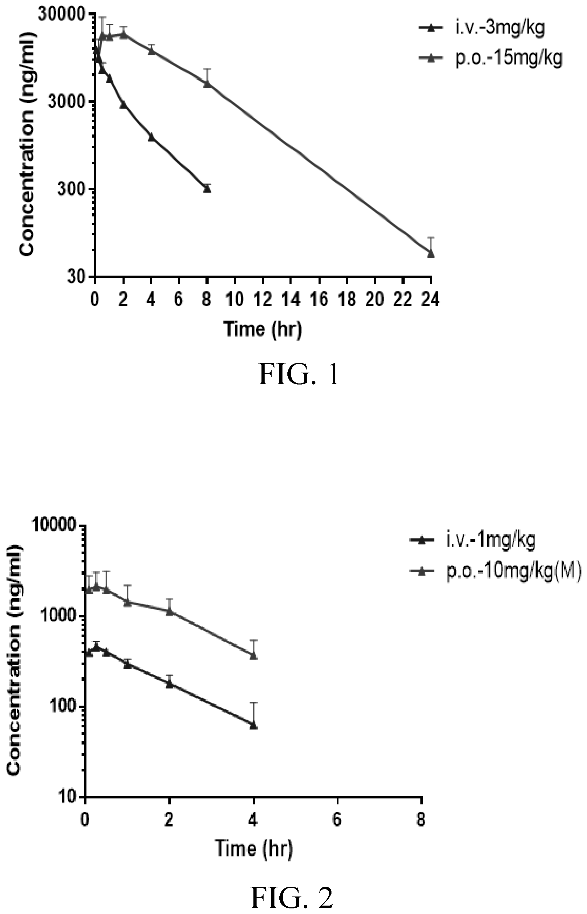

Twelve healthy adult female Sprague-Dawley (SD) rats (purchased from Beijing Vital River Laboratory Animal Technology Co., Ltd., China) with an average body weight of about 180-220 g were selected, and divided into four groups (n=3 per group). Two groups were administered via tail vein injection, while the other two groups were administered via oral gavage. Compound 1b was dissolved in a vehicle containing 5% DMSO, 30% PEG400, and 65% H₂O, and Debio-1450 was dissolved in physiological saline, both of which were formed into a stable clear solution for later use. Animals were fasted for 12 hours before administration and resumed normal feeding 2 hours post administration. Then, blood samples were collected through the jugular vein at the following time points: 0.083 h, 0.25 h, 0.5 h, 1 h, 2 h, 4 h, 8 h, 24 h (tail vein injection) and 0.25 h, 0.5 h, 1 h, 2 h, 4 h, 8 h, 24 h (oral gavage administration), respectively, with a blood collection volume of about 200-400 µL. Whole blood collected at each time point was immediately transferred into an anticoagulant tube containing K₂EDTA, then stored on ice in an insulated container. All samples were centrifuged within 15 minutes at 4600 r/min and 4°C for 5 minutes to separate plasma. The concentration of the compound in the plasma of each rat was determined using LC-MS/MS method, and the pharmacokinetic parameter was calculated from the drug concentration-time profile. The results are shown in Table 4, FIG. 1 and FIG.2.

**Table 4 Oral Pharmacokinetic Parameters of Compound 1b/Debio-1450 In Rats**

| **Compound Nos.** | **Dose (mg/Kg)** | **Tₘₐₓ (hr)** | **Cₘₐₓ (ng/mL)** | **T_{1/2} (hr)** | **AUC_{0-inf} (hr*ng/mL)** | **F (%)** |
|---|---|---|---|---|---|---|
| **1b** | 15 | 1.5 | 19, 645 | 2.57 | 110, 466 | 110 |
| **Debio-1450** | 10 | 0.67 | 2211 | 4.20 | 11, 539 | 97 |

The pharmacokinetic properties of the compound in the present disclosure have been evaluated through the above test experiments. The experimental results show that the compound in the present disclosure exhibits an excellent pharmacokinetic profile in rats after intravenous injection or oral administration, with higher Cₘₐₓ and greater AUC values compared to Debio-1450.

### Test Example 4

In this test experiment, the in vivo efficacy of the compound in mice was evaluated using a systemic infection model.

Ninety healthy adult male Balb/c mice (purchased from Beijing Vital River Laboratory Animal Technology Co., Ltd. , China) were selected, and divided into 10 groups, including one negative control group (9 mice, inoculated with bacteria and given vehicle), one positive control group (9 mice, vancomycin), one linezolid test group (9 mice), three test groups of high, medium, and low doses (3 ×9 mice) for compound 1a, three test groups of high, medium, and low doses (3 ×9 mice) for Debio-1450, and one test group for Debio-1452 (9 mice). All mice were acclimatized for 3-4 days before testing. The strain inoculated in this test example was methicillin-resistant *S. aureus* (NRS384), which was administered via tail vein injection at a dose of 1.0 × 10⁸ CFU/mouse. The compound was administered 0.5 h post inoculation with the strain. The compound 1a and Debio-1452 were formulated in a vehicle containing 5% DMSO, 30% PEG400, and 65% H₂O, while Debio-1450 was formulated in an isotonic glucose solution (freshly prepared) as the vehicle and was administered orally as a single dose. The specific dosing regimen is shown in Table 5:

**Table 5 Dosing Regimen for Systemic Infection Efficacy Model**

| **Group** | **Number of Animals** | **Vaccination Modeling** | **Treatment (Single-dose oral administration)** | **Pharmacodynamic indicators** |
|---|---|---|---|---|
| 1 | 9 | | Vehicle (Negative control group) | |
| 2 | 9 | | Vancomycin, 100 mg/Kg | |
| 3 | 9 | | Linezolid, 3 mg/Kg | |
| 4 | 9 | *S. aureus* NRS384, administered via tail vein injection | 1a, 0.3 mg/Kg | Survival rate of mice post inoculation |
| 5 | 9 | | 1a, 1.0 mg/Kg | |
| 6 | 9 | | 1a, 3.0 mg/Kg | |
| 7 | 9 | | Debio-1450, 0.3 mg/Kg | |
| 8 | 9 | | Debio-1450, 1.0 mg/Kg | |
| 9 | 9 | | Debio-1450, 3.0 mg/Kg | |
| 10 | 9 | | Debio-1452, 1.0 mg/Kg | |

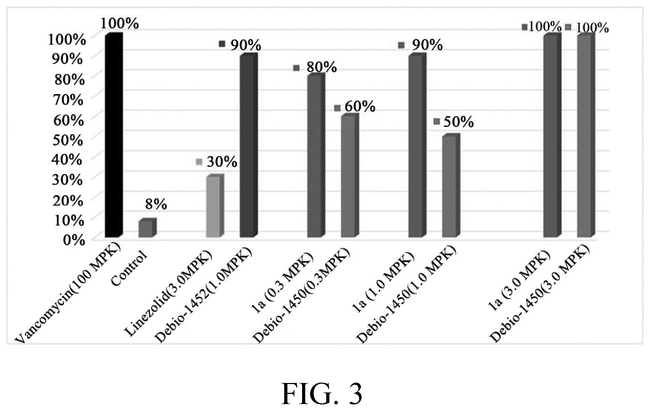

The in vivo efficacy of the compound in the present disclosure has been verified by the above experiments. The experimental results are shown in FIG. 3. After oral administration, the compound in the present disclosure shows a significant protective effect in mice compared to the negative control group, and the protective effect increases with the increase of dose. Compared with the reference compounds linezolid and Debio-1450, compound 1a shows a markedly superior efficacy to linezolid and better efficacy than Debio-1450.

The above examples are preferred embodiments of the present disclosure, but the embodiments of the present disclosure are not limited thereto. Any other changes, modifications, substitutions, combinations, or simplifications made without departing from the essence of protection and principles of the present disclosure shall be equivalent substitution methods and be included in the scope of protection of the present disclosure.

## Claims

1. A compound of Formula I, or a stereoisomer, a deuterated compound, an isotopically labeled derivative, a pharmaceutically acceptable salt, a prodrug, or a solvate thereof: 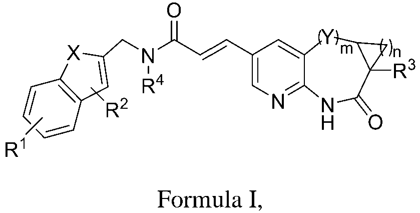
wherein X is selected from the group consisting of O, CR^{X1}R^{X2}, NR^{X1}, and S;
Y is selected from the group consisting of O, -CH₂-, NR^{Y}, -O-CH₂-, -CH₂-O-, -NR^{Y}-CH₂-, and -CH₂-NR^{Y}-;
R^{X1} and R^{X2} are each independently selected from the group consisting of hydrogen, -C₁₋₆ alkyl, and halogen-substituted -C₁₋₆ alkyl;
R^{Y} is selected from the group consisting of hydrogen, -C₁₋₆ alkyl, halogen-substituted -C₁₋₆ alkyl, -C₀₋₂ alkylene-C(O)R^{Y1}, -C₀₋₂ alkylene-C(O)NR^{Y1}R^{Y2}, -C₀₋₂ alkylene-NR^{Y1}R^{Y2}, -C₀₋₂ alkylene-NR^{Y1}C(O)R^{Y2}, -C₀₋₂ alkylene-(3-10-membered cycloalkyl), -C₀₋₂ alkylene-(3-10-membered heterocycloalkyl), -C₀₋₂ alkylene-(6-10-membered aromatic ring), and -C₀₋₂ alkylene-(5-10-membered heteroaromatic ring);
R^{Y1} and R^{Y2} are each independently selected from the group consisting of hydrogen, -C₁₋₆ alkyl, and halogen-substituted -C₁₋₆ alkyl;
R¹ is selected from the group consisting of hydrogen, halogen, cyano, nitro, -C₁₋₆ alkyl, halogen-substituted -C₁₋₆ alkyl, -C₀₋₂ alkylene-OR¹¹, -C₀₋₂ alkylene-C(O)R¹¹, -C₀₋₂ alkylene-C(O)NR¹¹R¹², -C₀₋₂ alkylene-NR¹¹R¹², -C₀₋₂ alkylene-NR¹¹C(O)R¹², -C₀₋₂ alkylene-(3-10-membered cycloalkyl), -C₀₋₂ alkylene-(3-10-membered heterocycloalkyl), -C₀₋₂ alkylene-(6-10-membered aromatic ring), and -C₀₋₂ alkylene-(5-10-membered heteroaromatic ring);
R¹¹ and R¹² are each independently selected from the group consisting of hydrogen, halogen, -C₁₋₆ alkyl, halogen-substituted -C₁₋₆ alkyl, -C₀₋₂ alkylene-(3-10-membered cycloalkyl), -C₀₋₂ alkylene-(3-10-membered heterocycloalkyl), -C₀₋₂ alkylene-(6-10-membered aromatic ring), and -C₀₋₂ alkylene-(5-10-membered heteroaromatic ring);
R² is selected from the group consisting of hydrogen, halogen, cyano, nitro, -C₁₋₆ alkyl, halogen-substituted -C₁₋₆ alkyl, -C₀₋₂ alkylene-OR²¹, -C₀₋₂ alkylene-C(O)R²¹, -C₀₋₂ alkylene-C(O)NR²¹R²², -C₀₋₂ alkylene-NR²¹R²², -C₀₋₂ alkylene-NR²¹C(O)R²², -C₀₋₂ alkylene-(3-10-membered cycloalkyl), -C₀₋₂ alkylene-(3-10-membered heterocycloalkyl), -C₀₋₂ alkylene-(6-10-membered aromatic ring), and -C₀₋₂ alkylene-(5-10-membered heteroaromatic ring);
R²¹ and R²² are each independently selected from the group consisting of hydrogen, halogen, -C₁₋₆ alkyl, halogen-substituted -C₁₋₆ alkyl, -C₀₋₂ alkylene-(3-10-membered cycloalkyl), and -C₀₋₂ alkylene-(3-10-membered heterocycloalkyl);
R³ is selected from the group consisting of hydrogen, halogen, cyano, nitro, hydroxy, -C₁₋₆ alkyl, halogen-substituted -C₁₋₆ alkyl, -C₀₋₂ alkylene-OR³¹, -C₀₋₂ alkylene-C(O)R³¹, -C₀₋₂ alkylene-C(O)NR³¹R³², -C₀₋₂ alkylene-NR³¹R³², -C₀₋₂ alkylene-NR³¹C(O)R³², -C₀₋₂ alkylene-(3-10-membered cycloalkyl), -C₀₋₂ alkylene-(3-10-membered heterocycloalkyl), -C₀₋₂ alkylene-(6-10-membered aromatic ring), and -C₀₋₂ alkylene-(5-10-membered heteroaromatic ring);
R³¹ and R³² are each independently selected from the group consisting of hydrogen, halogen, -C₁₋₆ alkyl, halogen-substituted -C₁₋₆ alkyl, -C₀₋₂ alkylene-NR³³R³⁴, -C₀₋₂ alkylene-(3-10-membered cycloalkyl), -C₀₋₂ alkylene-(3-10-membered heterocycloalkyl), -C₀₋₂ alkylene-(6-10-membered aromatic ring), and -C₀₋₂ alkylene-(5-10-membered heteroaromatic ring);
R³³ and R³⁴ are each independently selected from the group consisting of hydrogen, halogen, -C₁₋₆ alkyl, and halogen-substituted -C₁₋₆ alkyl;
R⁴ is selected from the group consisting of hydrogen, -C₁₋₆ alkyl, and halogen-substituted -C₁₋₆ alkyl;
m is 0, 1 or 2; and n is 1 or 2;
wherein C₀ means that the radical group is absent.

2. The compound of Formula I, or the stereoisomer, the deuterated compound, the isotopically labeled derivative, the pharmaceutically acceptable salt, the prodrug, or the solvate thereof of claim 1, wherein the compound of Formula I is represented by Formula II: 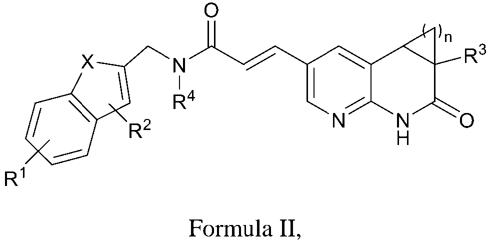 wherein R¹, R², R³, R⁴, X, and n are as defined in claim 1.

3. The compound of Formula I, or the stereoisomer, the deuterated compound, the isotopically labeled derivative, the pharmaceutically acceptable salt, the prodrug, or the solvate thereof of claim 1, wherein the compound of Formula I is represented by Formula III: 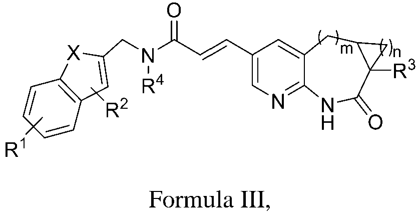 wherein R¹, R², R³, R⁴, X, m, and n are as defined in claim 1.

4. The compound of Formula I, or the stereoisomer, the deuterated compound, the isotopically labeled derivative, the pharmaceutically acceptable salt, the prodrug, or the solvate thereof of claim 1, wherein the compound of Formula I is represented by Formula IV: 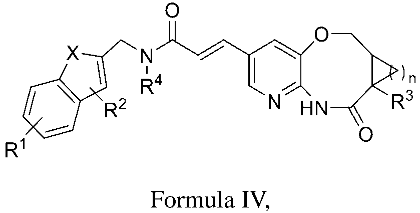 wherein R¹, R², R³, R⁴, X, and n are as defined in claim 1.

5. The compound of Formula I, or the stereoisomer, the deuterated compound, the isotopically labeled derivative, the pharmaceutically acceptable salt, the prodrug, or the solvate thereof of claim 1, wherein the compound of Formula I is represented by Formula V: 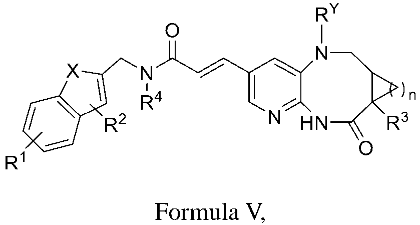 wherein R¹, R², R³, R⁴, R^{Y}, X, and n are as defined in claim 1.

6. The compound of Formula I, or the stereoisomer, the deuterated compound, the isotopically labeled derivative, the pharmaceutically acceptable salt, the prodrug, or the solvate thereof of claim 1, wherein R³ is selected from the group consisting of -C₀₋₂ alkylene-OR³¹, -C₀₋₂ alkylene-NR³¹R³², -C₀₋₂ alkylene-NR³¹C(O)R³², and -(3-6-membered heterocycloalkyl);
R³¹ and R³² are each independently selected from the group consisting of hydrogen, -C₁₋₃ alkyl, and -C₀₋₂ alkylene-NR³³R³⁴;
R³³ and R³⁴ are each independently selected from the group consisting of hydrogen, and -C₁₋₃ alkyl.

7. The compound of Formula I, or the stereoisomer, the deuterated compound, the isotopically labeled derivative, the pharmaceutically acceptable salt, the prodrug, or the solvate thereof of claim 1, wherein R³ is selected from the group consisting of -NH₂, 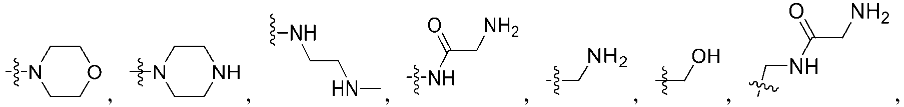 and

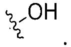

8. The compound of Formula I, or the stereoisomer, the deuterated compound, the isotopically labeled derivative, the pharmaceutically acceptable salt, the prodrug, or the solvate thereof of claim 1, wherein the compound of formula I is selected from the group consisting of:

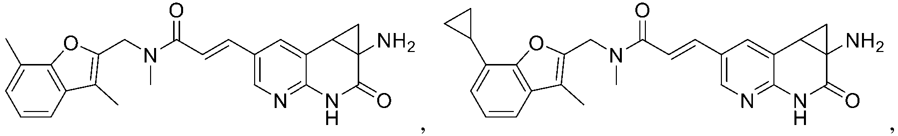

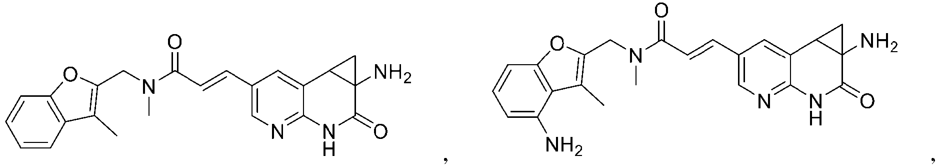

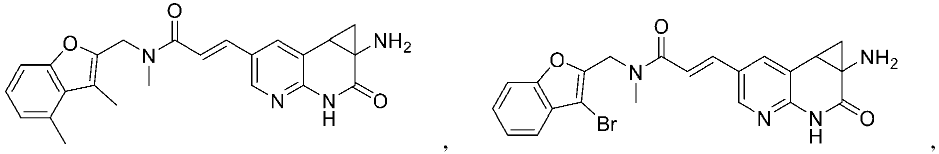

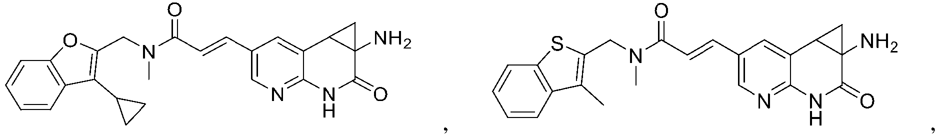

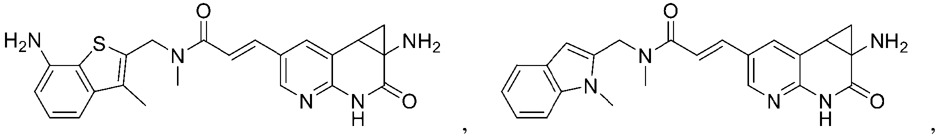

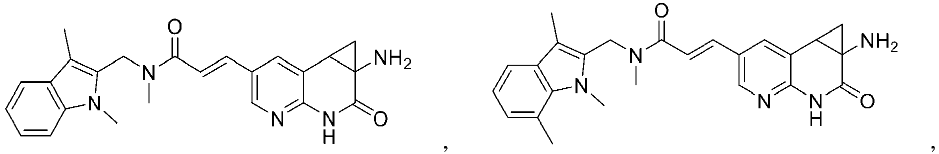

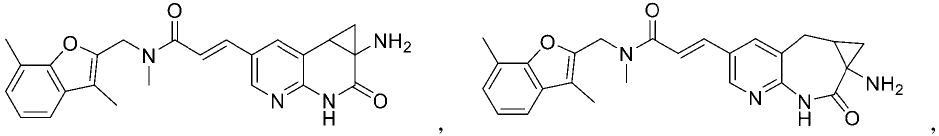

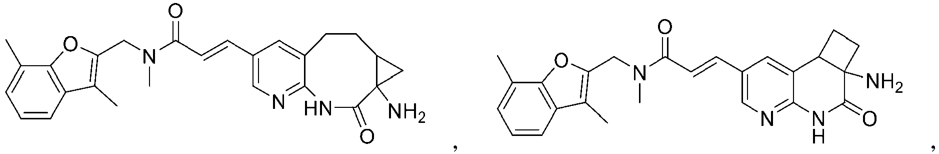

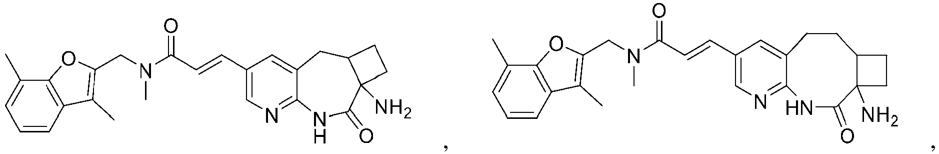

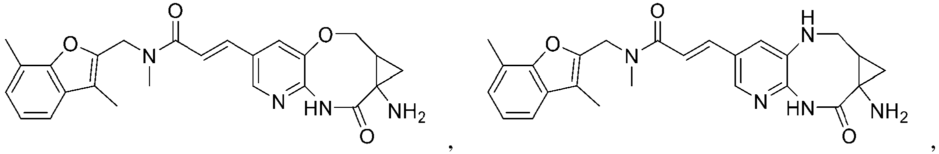

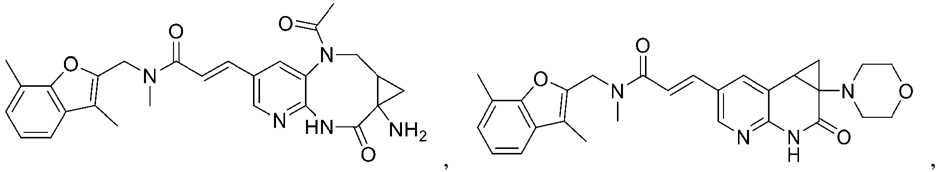

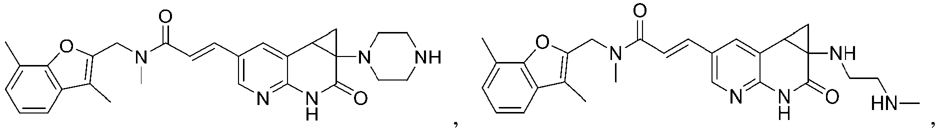

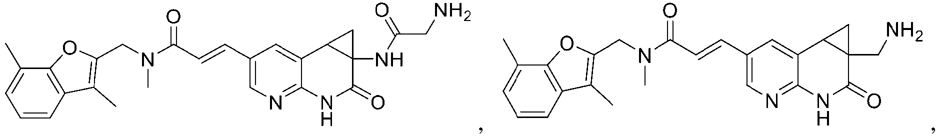

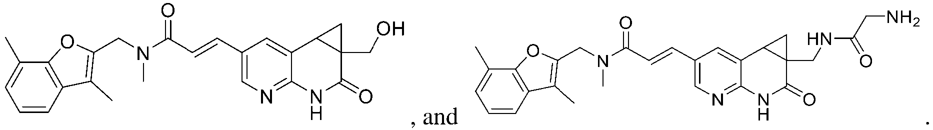

9. A pharmaceutical composition comprising the compound of Formula I, or the stereoisomer, the deuterated compound, the isotopically labeled derivative, the pharmaceutically acceptable salt, the prodrug, or the solvate thereof of any one of claims 1 to 8.

10. Use of the compound of Formula I, or the stereoisomer, the deuterated compound, the isotopically labeled derivative, the pharmaceutically acceptable salt, the prodrug, or the solvate thereof of any one of claims 1 to 8, or the pharmaceutical composition of claim 9 in the preparation of a drug for treating and/or preventing a FabI enzyme-mediated disease.
